(19) Europäisches Patentamt
European Patent Office
Office européen des brevets

(11) Veröffentlichungsnummer: **0 407 877 B1**

# (12) EUROPÄISCHE PATENTSCHRIFT

(45) Veröffentlichungstag der Patentschrift: **29.06.94**

(51) Int. Cl.5: **C07D 249/08**, C07D 233/56, A01N 43/653, A01N 43/50

(21) Anmeldenummer: **90112751.4**

(22) Anmeldetag: **04.07.90**

## (54) 1,2-Dihalogenazolylethanderivate und diese enthaltende Pflanzenschutzmittel.

(30) Priorität: **13.07.89 DE 3923151**

(43) Veröffentlichungstag der Anmeldung:
**16.01.91 Patentblatt 91/03**

(45) Bekanntmachung des Hinweises auf die Patenterteilung:
**29.06.94 Patentblatt 94/26**

(84) Benannte Vertragsstaaten:
**AT BE CH DE DK ES FR GB GR IT LI NL SE**

(56) Entgegenhaltungen:
**EP-A- 0 194 064**
**EP-A- 0 195 557**
**EP-A- 0 275 866**
**EP-A- 0 346 727**

(73) Patentinhaber: **BASF Aktiengesellschaft**
**Carl-Bosch-Strasse 38**
**D-67063 Ludwigshafen(DE)**

(72) Erfinder: **Seele, Rainer, Dr.**
**Leiblstrasse 3**
**D-6701 Fussgoenheim(DE)**
Erfinder: **Goetz, Norbert, Dr.**
**Schoefferstrasse 25**
**D-6520 Worms 1(DE)**
Erfinder: **Himmele, Walter, Dr.**
**Eichenweg 14**
**D-6909 Walldorf(DE)**
Erfinder: **Kober, Reiner, Dr.**
**Im Schlittweg 20**
**D-6701 Fussgoenheim(DE)**
Erfinder: **Rohr, Wolfgang, Dr.**
**In der Dreispitz 13**
**D-6706 Wachenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**
Erfinder: **Rademacher, Wilhelm, Dr.**
**Austrasse 1**
**D-6703 Limburgerhof(DE)**
Erfinder: **Jung, Johann, Prof.-Dr.**
**Hardenburgstrasse 19**
**D-6703 Limburgerhof(DE)**

EP 0 407 877 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolylverbindungen, Verfahren zu ihrer Herstellung und diese enthaltende Fungizide und Wachstumsregulatoren.

Es ist bekannt, Triazolderivate z.B. den 2,4'-Difluor-α-(1,2,4-triazol-1-yl-methyl)benzhydrylalkohol als Fungizid zu verwenden (EP-A-15756). Seine fungizide Wirkung ist jedoch ungenügend.

1-Azolyl-propan-derivate mit fungizider Wirkung sind in EP-A-195557 beschrieben.

Es wurde nun gefunden, daß 1,2-Dihalogenazolylethanderivate der allgemeinen Formel I

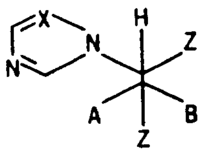

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,

Z den Rest Chlor oder Brom bedeutet,

X den Rest CH oder N bedeutet,

sowie deren für Pflanzen verträgliche Säureadditionssalze oder Metallkomplexe eine bessere fungizide Wirkung besitzen als bekannte Azolverbindungen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen.

Als fungizide und wachstumsregulierende Wirkstoffe können sowohl die einzelnen Enantiomeren oder Diastereomeren als auch deren Gemische verwendet werden.

A und B sind gleich oder verschieden und bedeuten beispielsweise 1-Naphthyl, 2-Naphthyl, p-Biphenyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, $C_1$-$C_4$-Alkoxyphenyl, 2-Methoxyphenyl, 3-Methoxyphenyl, 4-Methoxyphenyl, 2,4-Dimethoxyphenyl, $C_1$-$C_4$-Alkylphenyl,4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluoromethylphenyl, Halogen-$C_1$-$C_4$-alkylphenyl, 3-Trifluoromethylphenyl,4-Trifluoromethylphenyl, Pyridyl, 3-Pyridyl, Furyl, 2-Furyl, Thienyl, 2-Thienyl, 3-Thienyl, Isoxazolyl, 5-Isoxazolyl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der 1,2-Dihalogenazolylethanderivate (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die 1,2-Dihalogenazolylethanderivate mit entsprechenden Metallsalzen umsetzt.

Die Verbindungen der Formel I können z.B. hergestellt werden, indem man eine Verbindung der Formel II

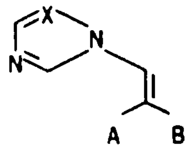

II

in welcher A, B und X die oben angegebene Bedeutung haben mit Chlor oder Brom in Gegenwart von Lewissäuren wie z.B. Zinkchlorid oder Zinkbromid umsetzt.

Die Reaktion erfolgt gegebenenfalls in Gegenwart eines Lösungs-oder Verdünnungsmittels bei Temperaturen zwischen -30 und 100 °C. Zu den bevorzugten Lösungsmitteln gehören z.B. Ester wie Essigsäureethylester, Essigsäuremethylester oder Essigsäurebutylester, Ether wie Tetrahydrofuran, Diethylester, Dimethoxyethan, Dioxan oder Diisopropylether und insbesondere Kohlenwasserstoffe und Chlorkohlenwasserstoffe wie Pentan, Hexan, Benzol, Methylenchlorid, Chloroform, Tetrachlorkohlenstoff, Dichlorethan oder entsprechende Gemische.

Als Lewissäuren kommen vorzugsweise Metallhalogenide wie z.B. Zinkchlorid, Zinkbromid, Zinnchlorid, Zinnbromid, Eisentribromid, Aluminiumtrichlorid, Titantetrachlorid in Frage.

Die Umsetzung wird im allgemeinen bei Temperaturen zwischen -30 °C und 100 °C, drucklos oder unter Druck, kontinuierlich oder diskontinuierlich durchgeführt.

Die Ausgangsverbindungen II können beispielsweise hergestellt werden, indem man eine Verbindung der Formel III

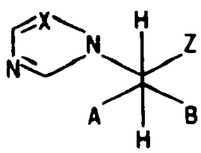

III

in welcher A, B, X und Z die oben angegebenen Bedeutungen haben, mit Basen umsetzt.

Die Reaktion erfolgt gegebenenfalls in Gegenwart eines Lösungs-oder Verdünnungsmittels unter Zusatz einer anorganischen oder organischen Base und gegebenenfalls unter Zusatz eines Reaktionsbeschleunigers bei Temperaturen zwischen 10 und 150 °C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid, Alkalicarbonate wie Natrium- und Caesiumcarbonat, Alkalihydride wie Lithium-, Natrium- und Kaliumhydrid, Alkaliamide wie Natrium- und Kaliumamid, ferner Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quarternäre Ammoniumsalze wie Tetrabutylammoniumbromid oder -jodid in Frage.

Die Verbindungen der Formel II können jedoch auch nach bekannten Verfahren (siehe EP-A-60 223) hergestellt werden.

Die Verbindungen der Formel III können hergestellt werden, indem man eine Verbindung der Formel IV

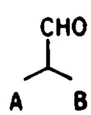

IV

in welcher A und B die oben angegebene Bedeutung haben, mit einer Verbindung der Formel V

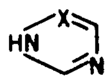

V

in welcher X die angegebene Bedeutung hat, in Gegenwart von entsprechenden Thionylhalogeniden ($SOZ_2$) zur Umsetzung bringt.

Die Umsetzung erfolgt gegebenenfalls in Gegenwart eines Lösungs-oder Verdünnungsmittels bei Temperaturen zwischen -30 und 80 °C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die Verbindungen der Formel IV lassen sich entsprechend allgemein bekannten Verfahren zur Aldehydsynthese (Houben-Weyl-Müller, Methoden der Organischen Chemie, Georg Thieme Verlag, Stuttgart 1983, Bd E3) herstellen.

Die Verbindungen der Formel I können außerdem hergestellt werden, indem man eine Verbindung der Formel

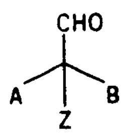

in welcher A, B und Z die oben angegebenen Bedeutungen habe, mit einer Verbindung der Formel

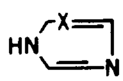

in welcher X die angegebene Bedeutung hat, in Gegenwart von Thionylhalogeniden ($SOZ_2$) zur Umsetzung bringt.

Die Umsetzung erfolgt gegebenenfalls in Gegenwart eines Lösungs- oder Verdünnungsmittels bei Temperaturen zwischen -30 °C und 80 °C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die folgenden Beispiele erläutern die Herstellung der Wirkstoffe.

I. Herstellung der Ausgangsstoffe

Vorschrift 1

1-Chlor-1-(1,2,4-triazol-1-yl)-2-(2-fluorphenyl)-2-(4-fluorphenyl)-ethan

Zu einer Lösung von 119,6 g Triazol in 500 ml Dichlormethan werden bei 0 °C 51,55 g Thionylchlorid zugesetzt. Nach beendeter Zugabe wird bei Raumtemperatur (20 °C) für 30 Minuten gerührt und anschließend 67 g 2-Fluorphenyl-4-fluorphenyl-acetaldehyd zugegeben. Nachdem das Reaktionsgemisch 12 Stunden bei Raumtemperatur rührte, wird der Lösung 300 ml Wasser zugesetzt und die organische Phase abgetrennt. Die verbleibende wässrige Phase wird zweimal mit Dichlormethan ausgeschüttelt und die gesammelten organischen Phasen zweimal mit gesättigter Natriumhydrogencarbonbat-Lösung gewaschen. Die abgetrennte organische Phase wird daraufhin über Natriumsulfat getrocknet und eingeengt, wobei 84,7 g (92 % 1-Chlor-1-(1,2,4-triazol-1-yl)-2-(2-fluorphenyl)-2-(4-fluor-phenyl)-ethan als 1:1-Diastereomerengemisch erhalten werden. Öl.

Vorschrift 2

1-(1,2,4-Triazol-1-yl)-2-(2-fluorphenyl)-2-(4-fluorphenyl)-ethen

Zu einer Lösung von 84,7 g 1-Chlor-1-(1,2,4-triazol-1-yl)-2-(2-fluorphenyl)-2-(4-fluorphenyl)-ethan in 500 ml Methanol werden 28,6 g Natriummethylat und 0,2 g Kaliumjodid gegeben. Nachdem das Reaktionsgemisch für eine Stunde unter Rückfluß erhitzt wurde, werden der Lösung 300 ml Wasser zugesetzt und mehrmals mit Methyl-tert.-butylether ausgeschüttelt. Die abgetrennte organische Phase wird zweimal mit Wasser gewaschen, daraufhin über Natriumsulfat getrocknet und eingeengt, wobei 67 g (89 %) 1-(1,2,4-Triazol-1-yl)-2-(2-fluorphenyl)-2-(4-fluorphenyl)-ethen erhalten werden. Öl.

II. Herstellung der Endprodukte

Beispiel 1

1,2-Dichlor-1-(1,2,4-triazol-1-yl)-2-(2-fluorphenyl)-2-(4-fluor-phenyl)-ethan (Wirkstoff Nr. 1)

Zu einer Lösung von 22,4 g 1-(1,2,4-Triazol-1-yl)-2-(2-fluorphenyl)-2-(4-fluorphenyl)-ethen in 80 ml Tetrachlorkohlenstoff werden 1,1 g Zinkchlorid gegeben und anschließend 8,4 g Chlor eingegast. Nachdem das Reaktionsgemisch zwei Stunden bei Raumtemperatur rührte, wird der entstandene Niederschlag abgesaugt, in Methylenchlorid aufgenommen, mehrmals mit gesättigter Natriumhydrogencarbonat-Lösung gewaschen, über Natriumsulfat getrocknet und eingeengt. Man erhält 17,8 g (63 %) 1,2-Dichlor-1-(1,2,4-triazol-1-yl)-2-(2-fluorphenyl)-2-(4-fluor-phenyl)-ethan als 1:1-Diastereomerengemisch.

Entsprechend Beispiel 1 können die in der Tabelle aufgeführten Verbindungen hergestellt werden.

Tabelle

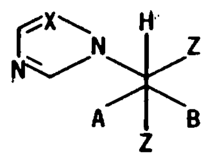

I

| Bsp | A | B | X | Z | Schmp./IR | Isomer* |
|---|---|---|---|---|---|---|
| 1 | 4-F-$C_6H_4$ | 2-F-$C_6H_4$ | N | Cl | 1604, 1507, 1237, 816 754 $cm^{-1}$ | $D_1:D_2=1:1$ |
| 2 | 4-F-$C_6H_4$ | 2-F-$C_6H_4$ | CH | Cl | | |
| 3 | 4-F-$C_6H_4$ | 2-F-$C_6H_4$ | N | Br | 1602, 1509, 1226, 836 760 $cm^{-1}$ | $D_1:D_2=1:1$ |
| 4 | 4-F-$C_6H_4$ | 2-F-$C_6H_4$ | CH | Br | | |
| 5 | 4-F-$C_6H_4$ | 3-F-$C_6H_4$ | N | Cl | | |
| 6 | 4-F-$C_6H_4$ | 4-F-$C_6H_4$ | N | Cl | 127-129°C | Enantiomerengemisch |
| 7 | 4-F-$C_6H_4$ | 4-F-$C_6H_4$ | CH | Cl | | |
| 8 | 4-F-$C_6H_4$ | 4-F-$C_6H_4$ | N | Br | | |
| 9 | 4-F-$C_6H_4$ | 4-F-$C_6H_4$ | CH | Br | | |
| 10 | 4-F-$C_6H_4$ | $C_6H_5$ | N | Cl | Harz | $D_1:D_2=1:1$ |
| 11 | 4-F-$C_6H_4$ | $C_6H_5$ | N | Br | | |
| 12 | 4-F-$C_6H_4$ | $C_6H_5$ | CH | Cl | | |
| 13 | 4-F-$C_6H_4$ | 2-Cl-$C_6H_4$ | N | Cl | 1506, 1277, 1236, 816, 750 $cm^{-1}$ | $D_1:D_2=1:1$ |
| 14 | 4-F-$C_6H_4$ | 2-Cl-$C_6H_4$ | CH | Cl | | |
| 15 | 4-F-$C_6H_4$ | 2-Cl-$C_6H_4$ | N | Br | | |
| 16 | 4-F-$C_6H_4$ | 2-Cl-$C_6H_4$ | CH | Br | | |

Forts. Tab.

| Bsp | A | B | X | Z | Schmp./IR | Isomer* |
|---|---|---|---|---|---|---|
| 17 | 4-F-$C_6H_4$ | 3-Cl-$C_6H_4$ | N | Cl | | |
| 18 | 4-F-$C_6H_4$ | 4-Cl-$C_6H_4$ | N | Cl | | |
| 19 | 4-F-$C_6H_4$ | 4-Cl-$C_6H_4$ | N | Br | | |
| 20 | 4-F-$C_6H_4$ | 4-Cl-$C_6H_4$ | CH | Cl | | |
| 21 | 4-F-$C_6H_4$ | 2-Br-$C_6H_4$ | N | Cl | | |
| 22 | 4-F-$C_6H_4$ | p-Biphenyl | N | Cl | | |
| 23 | 4-F-$C_6H_4$ | 2-Naphthyl | N | Cl | | |
| 24 | 4-F-$C_6H_4$ | 1-Naphthyl | N | Cl | | |
| 25 | 4-F-$C_6H_4$ | 2-$CH_3$-$C_6H_4$ | N | Cl | | |
| 26 | 4-F-$C_6H_4$ | 2-$CH_3$-$C_6H_4$ | N | Br | | |
| 27 | 4-F-$C_6H_4$ | 2-$CH_3$-$C_6H_4$ | CH | Cl | | |
| 28 | 4-F-$C_6H_4$ | 4-$CH_3$-$C_6H_4$ | N | Cl | | |
| 29 | 4-F-$C_6H_4$ | 2,4-di-$CH_3$-$C_6H_3$ | N | Cl | | |
| 30 | 4-F-$C_6H_4$ | 2-$CF_3$-$C_6H_4$ | N | Cl | | |
| 31 | 4-F-$C_6H_4$ | 2-$CF_3$-$C_6H_4$ | N | Br | | |
| 32 | 4-F-$C_6H_4$ | 2-$CF_3$-$C_6H_4$ | CH | Cl | | |
| 33 | 4-F-$C_6H_4$ | 3-$CF_3$-$C_6H_4$ | N | Cl | | |
| 34 | 4-F-$C_6H_4$ | 4-$CF_3$-$C_6H_4$ | N | Cl | | |
| 35 | 4-F-$C_6H_4$ | 4-$CF_3$-$C_6H_4$ | N | Br | | |
| 36 | 4-F-$C_6H_4$ | 4-$CF_3$-$C_6H_4$ | CH | Cl | | |
| 37 | 4-F-$C_6H_4$ | 3-$NO_2$-$C_6H_4$ | N | Cl | | |
| 38 | 4-F-$C_6H_4$ | 3-$NH_2$-$C_6H_4$ | N | Cl | | |
| 39 | 4-F-$C_6H_4$ | 2-$OCH_3$-$C_6H_4$ | N | Cl | | |
| 40 | 4-F-$C_6H_4$ | 2-$OCH_3$-$C_6H_4$ | N | Br | | |
| 41 | 4-F-$C_6H_4$ | 2-$OCH_3$-$C_6H_4$ | CH | Cl | | |

Forts. Tab.

| Bsp | A | B | X | Z | Schmp./IR | Isomer* |
|---|---|---|---|---|---|---|
| 42 | 4-F-$C_6H_4$ | 3-$OCH_3$-$C_6H_4$ | N | Cl | | |
| 43 | 4-F-$C_6H_4$ | 3-$OCH_3$-$C_6H_4$ | N | Br | | |
| 44 | 4-F-$C_6H_4$ | 4-$OCH_3$-$C_6H_4$ | N | Cl | | |
| 45 | 4-F-$C_6H_4$ | 4-$OCH_3$-$C_6H_4$ | N | Br | | |
| 46 | 4-F-$C_6H_4$ | 4-$OCH_3$-$C_6H_4$ | CH | Cl | | |
| 47 | 4-F-$C_6H_4$ | 2-Pyridyl | N | Cl | | |
| 48 | 4-F-$C_6H_4$ | 3-Pyridyl | N | Cl | | |
| 49 | 4-F-$C_6H_4$ | 4-Pyridyl | N | Cl | | |
| 50 | 4-F-$C_6H_4$ | 2-Thienyl | N | Cl | | |
| 51 | 4-F-$C_6H_4$ | 3-Thienyl | N | Cl | | |
| 52 | 4-F-$C_6H_4$ | 2-Furyl | N | Cl | | |
| 53 | 4-F-$C_6H_4$ | 5-Isoxazolyl | N | Cl | | |
| 54 | $C_6H_5$ | 2-F-$C_6H_4$ | N | Cl | | |
| 55 | $C_6H_5$ | $C_6H_5$ | N | Cl | | |
| 56 | $C_6H_5$ | 2-Cl-$C_6H_4$ | N | Cl | 1502, 1277, 1133, 752 $cm^{-1}$ | $D_1$:$D_2$=1:1 |
| 57 | $C_6H_5$ | 4-Cl-$C_6H_4$ | N | Cl | 1494, 1276, 1096, 1014 756, 701 $cm^{-1}$ | $D_1$:$D_2$=1:1 |
| 58 | $C_6H_5$ | 2-Br-$C_6H_4$ | N | Cl | | |
| 59 | $C_6H_5$ | 4-Br-$C_6H_4$ | N | Cl | | |
| 60 | $C_6H_5$ | 2-$CH_3$-$C_6H_4$ | N | Cl | | |
| 61 | $C_6H_5$ | 4-$CH_3$-$C_6H_4$ | N | Cl | | |
| 62 | $C_6H_5$ | 4-tert.-$C_4H_9$-$C_6H_4$ | N | Cl | | |
| 63 | $C_6H_5$ | 2-$CF_3$-$C_6H_4$ | N | Cl | | |
| 64 | $C_6H_5$ | 4-$CF_3$-$C_6H_4$ | N | Cl | | |
| 65 | $C_6H_5$ | 2-$OCH_3$-$C_6H_4$ | N | Cl | | |

Forts. Tab.

| Bsp | A | B | X | Z | Schmp./IR | Isomer* |
|---|---|---|---|---|---|---|
| 66 | $C_6H_5$ | 4-$OCH_3$-$C_6H_4$ | N | Cl | | |
| 67 | $C_6H_5$ | 2-Naphthyl | N | Cl | | |
| 68 | $C_6H_5$ | 2-Pyridyl | N | Cl | | |
| 69 | $C_6H_5$ | 3-Pyridyl | N | Cl | | |
| 70 | $C_6H_5$ | 2-Thienyl | N | Cl | | |
| 71 | $C_6H_5$ | 3-Thienyl | N | Cl | | |
| 72 | 4-Cl-$C_6H_4$ | 2-F-$C_6H_4$ | N | Cl | | |
| 73 | 4-Cl-$C_6H_4$ | 2-Cl-$C_6H_4$ | N | Cl | | |
| 74 | 4-Cl-$C_6H_4$ | 4-Cl-$C_6H_4$ | N | Cl | 1502,1490,831, 764 $cm^{-1}$ | Enantiomeren-gemisch |
| 75 | 4-Cl-$C_6H_4$ | 2-Br-$C_6H_4$ | N | Cl | | |
| 76 | 4-Cl-$C_6H_4$ | 4-Br-$C_6H_4$ | N | Cl | | |
| 77 | 4-Cl-$C_6H_4$ | 2-$CH_3$-$C_6H_4$ | N | Cl | | |
| 78 | 4-Cl-$C_6H_4$ | 4-$CH_3$-$C_6H_4$ | N | Cl | | |
| 79 | 4-Cl-$C_6H_4$ | 2-$CF_3$-$C_6H_4$ | N | Cl | | |
| 80 | 4-Cl-$C_6H_4$ | 4-$CF_3$-$C_6H_4$ | N | Cl | | |
| 81 | 4-Cl-$C_6H_4$ | 2-$OCH_3$-$C_6H_4$ | N | Cl | | |
| 82 | 4-Cl-$C_6H_4$ | 4-$OCH_3$-$C_6H_4$ | N | Cl | | |
| 83 | 4-Br-$C_6H_4$ | 2-F-$C_6H_4$ | N | Cl | | |
| 84 | 4-Br-$C_6H_4$ | 2-Cl-$C_6H_4$ | N | Cl | | |
| 85 | 4-Br-$C_6H_4$ | 2-$CH_3$-$C_6H_4$ | N | Cl | | |
| 86 | 4-Br-$C_6H_4$ | 2-$CF_3$-$C_6H_4$ | N | Cl | | |
| 87 | 4-Br-$C_6H_4$ | 4-$CF_3$-$C_6H_4$ | N | Cl | | |
| 88 | 4-Br-$C_6H_4$ | 2-$OCH_3$-$C_6H_4$ | N | Cl | | |
| 89 | 4-Br-$C_6H_4$ | 4-$OCH_3$-$C_6H_4$ | N | Cl | | |

Forts. Tab.

| Bsp | A | B | X | Z | Schmp./IR | Isomer* |
|---|---|---|---|---|---|---|
| 90 | $4-CH_3-C_6H_4$ | $2-Cl-C_6H_4$ | N | Cl | | |
| 91 | $4-CH_3-C_6H_4$ | $2-F-C_6H_4$ | N | Cl | | |
| 92 | $4-CH_3-C_6H_4$ | $2-CH_3-C_6H_4$ | N | Cl | | |
| 93 | $4-CH_3-C_6H_4$ | $4-CH_3-C_6H_4$ | N | Cl | | |
| 94 | $4-CH_3-C_6H_4$ | $2-CF_3-C_6H_4$ | N | Cl | | |
| 95 | $4-CH_3-C_6H_4$ | $4-CF_3-C_6H_4$ | N | Cl | | |
| 96 | $4-CH_3-C_6H_4$ | $2-OCH_3-C_6H_4$ | N | Cl | | |
| 97 | $4-CH_3-C_6H_4$ | $4-OCH_3-C_6H_4$ | N | Cl | | |
| 98 | $4-CF_3-C_6H_4$ | $2-Cl-C_6H_4$ | N | Cl | | |
| 99 | $4-CF_3-C_6H_4$ | $2-F-C_6H_4$ | N | Cl | | |
| 100 | $4-CF_3-C_6H_4$ | $2-CH_3-C_6H_4$ | N | Cl | | |
| 101 | $4-CF_3-C_6H_4$ | $2-CF_3-C_6H_4$ | N | Cl | | |
| 102 | $4-CF_3-C_6H_4$ | $2-OCH_3-C_6H_4$ | N | Cl | | |
| 103 | $4-OCH_3-C_6H_4$ | $2-Cl-C_6H_4$ | N | Cl | | |
| 104 | $4-OCH_3-C_6H_4$ | $2-F-C_6H_4$ | N | Cl | | |
| 105 | $4-OCH_3-C_6H_4$ | $2-CH_3-C_6H_4$ | N | Cl | | |
| 106 | $4-OCH_3-C_6H_4$ | $2-CF_3-C_6H_4$ | N | Cl | | |
| 107 | $4-OCH_3-C_6H_4$ | $2-OCH_3-C_6H_4$ | N | Cl | | |
| 108 | $4-OCH_3-C_6H_4$ | $4-OCH_3-C_6H_4$ | N | Cl | | |
| 109 | $2-F-C_6H_4$ | $2-Cl-C_6H_4$ | N | Cl | | |
| 110 | $2-F-C_6H_4$ | $2-F-C_6H_4$ | N | Cl | | |
| 111 | $2-F-C_6H_4$ | $2-CH_3-C_6H_4$ | N | Cl | | |
| 112 | $2-Cl-C_6H_4$ | $2-CH_3-C_6H_4$ | N | Cl | | |
| 113 | $2-Cl-C_6H_4$ | $2-CF_3-C_6H_4$ | N | Cl | | |
| 114 | $2-Cl-C_6H_4$ | $2-OCH_3-C_6H_4$ | N | Cl | | |

* $D_1:D_2$ = Verhältnis der gebildeten Diastereomeren

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,

Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Aufwandmengen der fungiziden Mittel liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii.

Die neuen Verbindungen können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von klimatischen Faktoren, z.B. Temperatur, Niederschlagsmenge, außerdem auch Tageslänge und Lichtintensität,

e) von der Bodenbeschaffenheit (einschließlich Düngung),

f) von der Formulierung bzw. Anwendungsform des WIrkstoffs und

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Von praktischer Bedeutung ist auch die Reduzierung des vegetativen Wachstums bei Obstbäumen und anderen Gehölzen, da so Schnittkosten eingespart werden können.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung der neuen Verbindungen kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den neuen Verbindungen läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die

Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Wirkstoffe der Formel I können Ertragssteigerungen durch Eingriffe in den pflanzlichen Stoffwechsel bzw. durch Förderung oder Hemmung des vegetativen und/oder des generativen Wachstums verursachen.

C. Mit den Verbindungen der Formel I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen, z.B. Baumwolle, wesentlich.

D. Mit den Wirkstoffen kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Wirkstoffe kann die Aufwandmenge als Wachstumsregulatoren stark variiert werden. Bei der Saatgutbehandlung werden im allgemeinen Wirkstoffmengen von 0,001 bis 50 g je Kilogramm Saatgut, vorzugsweise 0,005 bis 0,5 g, benötigt. Für die Blatt- und Bodenbehandlung sind im allgemeinen Gaben von 0,01 bis 10 kg/ha, bevorzugt 0,05 bis 1 kg/ha ausreichend.

Die Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylole, Toluol), chlorierte Aromaten (z.B. Chlorbenzole), Paraffine (z.B. Erdölfraktionen), Alkohole (z.B. Methanol, Butanol), Amine (z.B. Ethanolamin), N,N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin-Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N,N-Dimethylformamid oder N-Methylpyrrolidon.

Die fungiziden und wachstumsregulierenden Mittel enthalten im allgemeinen zwischen 0,1 und 95, vorzugsweise zwischen 0,5 und 90 Gew.% Wirkstoff.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 13 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 13 werden in einer Mischung gelöst, die aus 80 Gew.-Teilen Xylol, 10 Gew.-Teilen des Anlagerungsproduktes von 8 bis 10 Mol Ethylenoxid an 1 Mol ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 13 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 13 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 13 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 13 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 13 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 13 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 13 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 2,4'-Difluor-α-(1,2,4-triazol-1-yl-methyl)-benzhydrylalkohol (A)
- bekannt aus EP-A-15756 - benutzt.

Anwendungsbeispiel

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 bis 22 °C und 70 % relativer Luftfeuchtigkeit für weitere 5 Tage kultiviert.

Dann wurde das Ausmaß der Schädigung der Blätter ermittelt.

Das Ergebnis zeigt, daß der Wirkstoff 13 bei der Anwendung als 0,0125 %ige (Gew.-%) Spritzbrühe eine bessere fungizide Wirkung zeigt (100 %) als der bekannte Vergleichswirkstoff A (80 %).

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen (ca. 12,5 cm Durchmesser; Volumen ca. 500 ml) angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen:

Vergleichssubstanz:

CCC = 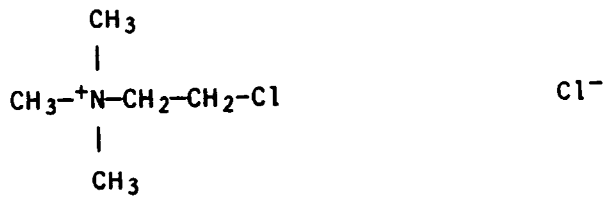

Tabelle 1

Sommerraps "Petranova"

Vorauflauf-Bodenbehandlung

| Wirkstoff Nr. | Konz. mg Wirkstoff/Gefäß | Wuchshöhen relativ |
|---|---|---|
| unbehandelt | - | 100 |
| CCC | 6 | 95,9 |
| 6 | 6 | 91,3 |
| 10 | 6 | 88,7 |

Tabelle 2

Sommerraps "Petranova"

Nachauflauf-Blattbehandlung

| Wirkstoff Nr. | Konz. mg Wirkstoff/Gefäß | Wuchshöhen relativ |
|---|---|---|
| unbehandelt | - | 100 |
| CCC | 6 | 92,2 |
| 1 | 6 | 88,3 |
| 10 | 6 | 84,4 |
| 74 | 6 | 75,6 |

## Patentansprüche

**1.** 1,2-Dihalogenazolylethanderivate der allgemeinen Formel I

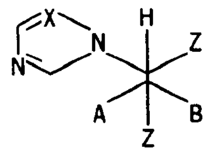

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,

Z den Rest Chlor oder Brom bedeutet,

X den Rest CH oder N bedeutet,

sowie deren für Pflanzen verträgliche Säureadditionssalze und Metallkomplexe.

**2.** Verfahren zur Herstellung der 1,2-Dihalogenazolylethanderivate der Formel I gemäß Anspruch 1, dadurch gekennzeichnet, daß man eine Verbindung der Formel II

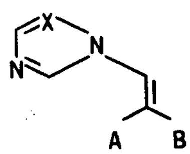

II

in welcher A, B und X die oben angegebenen Bedeutungen haben, mit Chlor oder Brom in Gegenwart von Lewissäuren zur Umsetzung bringt und die so erhaltenen Verbindungen gegebenenfalls in ihre für Pflanzen verträglichen Säureadditionssalze oder Metallkomplexe überführt.

**3.** Verfahren zur Herstellung der 1,2-Dihalogenazolylethanderivate der Formel I gemäß Anspruch 1, dadurch gekennzeichnet, daß man eine Verbindung der Formel

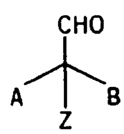

in welcher A, B und Z die oben angegebenen Bedeutungen haben, mit einer Verbindung der Formel

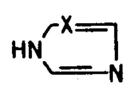

in welcher X die angegebene Bedeutung hat, in Gegenwart von Thionylhalogeniden ($SOZ_2$) zur Umsetzung bringt.

**4.** Fungizides Mittel, enthaltend einen Trägerstoff und eine fungizid wirksame Menge eines 1,2-Dihalogenazolylethanderivat der allgemeinen Formel I

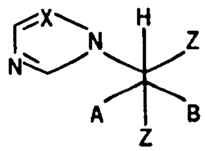

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,

Z den Rest Chlor oder Brom bedeutet,

X den Rest CH oder N bedeutet,

oder dessen für Pflanzen verträgliches Säureadditionssalz oder Metallkomplex.

**5.** Verfahren zur Bekämpfung von Pilzen, dadurch gekennzeichnet, daß man eine fungizid wirksame Menge eines 1,2-Dihalogenazolylethanderivates der Formel I

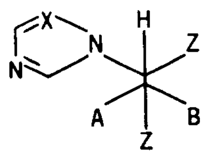

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,

Z den Rest Chlor oder Brom bedeutet,

X den Rest CH oder N bedeutet,

oder dessen für Pflanzen verträgliches Säureadditionssalz oder Metallkomplex auf die Pilze oder auf durch Pilzbefall bedrohte Materialien, Flächen, Pflanzen und Saatgüter einwirken läßt.

**6.** Wachstumsregulatorisches Mittel, enthaltend einen Trägerstoff und eine wachstumsregulierend wirksame Menge eines 1,2-Dihalogenazolylethanderivats der Formel I

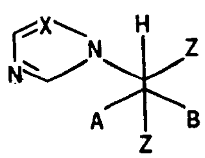

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,

Z den Rest Chlor oder Brom bedeutet,

X den Rest CH oder N bedeutet,

oder dessen für Pflanzen verträgliches Säureadditionssalz.

**7.** Verfahren zur Regulierung des Pflanzenwachstums, dadurch gekennzeichnet, daß man eine wachstumsregulierend wirksame Menge eines 1,2-Dihalogenazolylethanderivates der Formel I

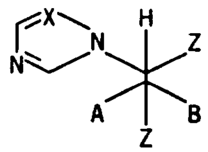

I

in welcher

A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,

Z den Rest Chlor oder Brom bedeutet,

X den Rest CH oder N bedeutet,

oder dessen für Pflanzen verträgliches Säureadditionssalz auf Kulturpflanzen oder auf den Boden einwirken läßt.

**8.** Verbindung der Formel I gemäß Anspruch 1, dadurch gekennzeichnet, daß

A 4-Fluorphenyl,

B 2-Chlorphenyl,

X N und Z Chlor bedeutet.

**Claims**

**1.** A 1,2-dihaloazolylethane derivative of the general formula I

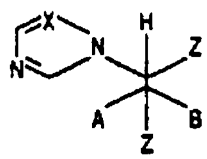

I

where A and B are identical or different and each is phenyl, biphenyl, naphthyl or hetaryl, and these radicals may be monosubstituted to trisubstituted by halogen, nitro, phenoxy, amino, alkyl, alkoxy or haloalkyl, each of 1 to 4 carbon atoms, Z is chlorine or bromine, and X is CH or N, or a plant-tolerated acid addition salt or metal complex thereof.

**2.** A process for the preparation of a 1,2-dihaloazolylethane derivative of the formula I as claimed in claim 1, which comprises reacting a compound of the formula II

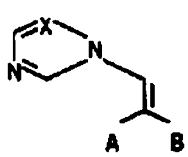

II

where A, B and X have the abovementioned meanings, with chlorine or bromine in the presence of a Lewis acid and, if appropriate, converting the resulting compound into its plant-tolerated acid addition salt or metal complex.

**3.** A process for the preparation of a 1,2-dihaloazolylethane derivative of the formula I as claimed in claim 1, which comprises reacting a compound of the formula

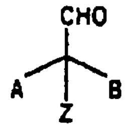

where A, B and Z have the abovementioned meanings, with a compound of the formula

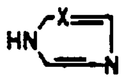

where X has the abovementioned meanings, in the presence of a thionyl halide ($SOZ_2$).

**4.** A fungicide containing a carrier and a fungicidally effective amount of a 1,2-dihaloazolylethane derivative of the general formula I

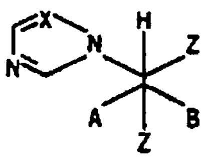

I

where A and B are identical or different and each is phenyl, biphenyl, naphthyl or hetaryl, and these radicals may be monosubstituted to trisubstituted by halogen, nitro, phenoxy, amino, alkyl, alkoxy or haloalkyl, each of 1 to 4 carbon atoms, Z is chlorine or bromine, and X is CH or N, or a plant-tolerated acid addition salt or metal complex thereof.

**5.** A method for controlling fungi, wherein a fungicidally effective amount of a 1,2-dihaloazolylethane derivative of the formula I

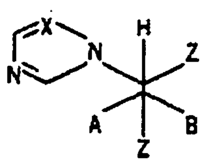

I

where A and B are identical or different and each is phenyl, biphenyl, naphthyl or hetaryl, and these radicals may be monosubstituted to trisubstituted by halogen, nitro, phenoxy, amino, alkyl, alkoxy or haloalkyl, each of 1 to 4 carbon atoms, Z is chlorine or bromine, and X is CH or N, or a plant-tolerated acid additon salt or metal complex thereof, is allowed to act on the fungi or on materials, areas, plants or seed threatened by fungal attack.

**6.** A growth regulator containing a carrier and a growth-regulating amount of a 1,2-dihaloazolylethane deivative of the formula I

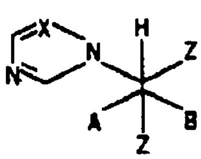

I

where A and B are identical or different and each is phenyl, biphenyl, naphthyl or hetaryl, and these radicals may be monosubstituted to trisubstituted by halogen, nitro, phenoxy, amino, alkyl, alkoxy or

haloalkyl, each of 1 to 4 carbon atoms, Z is chlorine or bromine, and X is CH or N, or a plant-tolerated acid additon salt thereof.

7. A method for regulating plant growth, wherein a growth-regulating amount of a 1,2-dihaloazolylethane derivative of the formula I

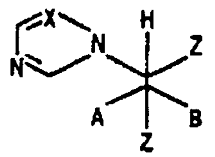

I

where A and B are identical or different and each is phenyl, biphenyl, naphthyl or hetaryl, and these radicals may be monosubstituted to trisubstituted by halogen, nitro, phenoxy, amino, alkyl, alkoxy or haloalkyl, each of 1 to 4 carbon atoms, Z is chlorine or bromine, and X is CH or N, or a plant-tolerated acid additon salt thereof, is allowed to act on crops or on the soil.

8. A compound of the formula I as claimed in claim 1, wherein A is 4-fluorophenyl, B is 2-chlorophenyl, X is N and Z is chlorine.

**Revendications**

1. Dérivés de 1,2-dihalogenazolylether de formule I

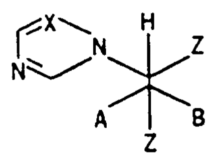

I

dans laquelle
A et B sont identiques ou différents et représentent phenyle, biphenyle, naphtyle ou hetaryle, ces restes pouvant être substitués de une à trois fois par halogène, nitro, phenoxy, amino, alkyle, alcoxy ou halogenalkyle ayant chacun 1 à 4 atomes C
Z représente chlore ou brome
X représente CH ou N
ou leurs sels d'addition d'acide ou complexes métalliques acceptables pour les plantes.

2. Procédé de préparation des dérivés de 1,2-dihalogenazolylether de formule I selon la revendication 1, caractérisé que l'on met à réagir, en présence d'acide de Lewis, un composé de formule II

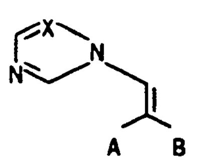

II

dans laquelle A, B et X ont les significations sus-indiquées avec du chlore ou du brome et l'on transforme eventuellement les composés ainsi obtenus, en leurs sels d'addition d'acide ou complexes métalliques acceptables pour les plantes.

3. Procédé de préparation des dérivés de 1,2-dihalogenazolylether de formule I selon la revendication 1, caractérisé en ce que l'on met à réagir, en présence d'halogenures de thionyle ($SOZ_2$), un composé de

la formule

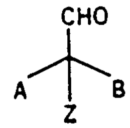

dans laquelle A, B et Z ont les significations sus-indiquées avec un composé de la formule

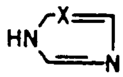

dans laquelle X a la signification indiquée,

**4.** Agent fongicide, contenant un support et une quantité efficace comme fongicide d'un dérivé de 1,2-dihalogenazolylether de formule I

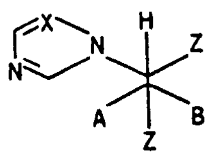

I

dans laquelle

| | |
|---|---|
| A et B | sont identiques ou différents et représentent phenyle, biphenyle, naphtyle ou hetaryle, ces restes pouvant être substitués de une à trois fois par halogène, nitro, phenoxy, amino, alkyle, alcoxy ou halogenalkyle ayant chacun 1 à 4 atomes C |
| Z | représente chlore ou brome |
| X | représente CH ou N |

ou ses sels d'addition d'acide ou complexes métalliques acceptables pour les plantes.

**5.** Procédé pour lutter contre les champignons, caractérisé en ce que l'on fait agir sur les champignons ou sur les matériaux, surfaces, plantes et semences, menacés d'une attaque par les champignons, une quantité efficace comme fongicide d'un dérivé de 1,2-dihalogenazolylether de la formule I

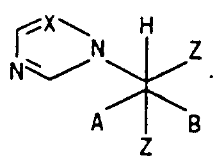

I

dans laquelle

| | |
|---|---|
| A et B | sont identiques ou différents et représentent phenyle, biphenyle, naphtyle ou hetaryle, ces restes pouvant être substitués de une à trois fois par halogène, nitro, phenoxy, amino, alkyle, alcoxy ou halogenalkyle ayant chacun 1 à 4 atomes C |
| Z | représente chlore ou brome |
| X | représente CH ou N |

ou ses sels d'addition d'acide ou complexes métalliques acceptables pour les plantes.

**6.** Agent régulateur de la croissance contenant un support et une quantité efficace comme fongicide d'un dérivé de 1,2-dihalogenazolylether de la formule I

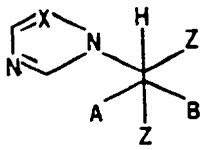

I

dans laquelle

A et B sont identiques ou différents et représentent phenyle, biphenyle, naphtyle ou hetaryle, ces restes pouvant être substitués de une à trois fois par halogène, nitro, phenoxy, amino, alkyle, alcoxy ou halogenalkyle ayant chacun 1 à 4 atomes C

Z représente chlore ou brome

X représente CH ou N

ou ses sels d'addition d'acide ou complexes métalliques acceptables pour les plantes.

**7.** Procédé pour la régularisation de la croissance des plantes, caractérisé en que l'on fait agir sur les plantes de culture ou sur le sol une quantité efficace pour régulariser la croissance d'un dérivé de 1,2-dihalogenazolylether de la formule I

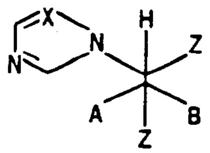

I

dans laquelle

A et B sont identiques ou différents et représentent phenyle, biphenyle, naphtyle ou hetaryle, ces restes pouvant être substitués de une à trois fois par halogène, nitro, phenoxy, amino, alkyle, alcoxy ou halogenalkyle ayant chacun 1 à 4 atomes C

Z représente chlore ou brome

X représente CH ou N

ou ses sels d'addition d'acide ou complexes métalliques acceptables pour les plantes.

**8.** Composé de formule I selon la revendication 1, caractérisé en que

A représente 4-fluorophenyle,

B représente 2-chlorophenyle,

X représente N et Z chlore